# EUROPEAN PATENT SPECIFICATION

(11) **EP 0 839 528 B1**
(45) Date of publication and mention of the grant of the patent: **14.03.2001**
(21) Application number: 96117324.2
(22) Date of filing: 29.10.1996
(51) Int. Cl.: A61K 31/195, A61K 47/40

(54) **Mouth-soluble compositions containing N-acetylcysteine and cyclodextrin**
Mundlösliche Zusammensetzungen die N-Acetylcystein und Cyclodextrin enthalten
Compositions solubles dans la bouche contenant de la N-acetyl-cysteine et de la cyclodextrine

(43) Date of publication of application: 06.05.1998
(73) Proprietor: STAROIL LIMITED, British Virgin Islands (GB)
(72) Inventor: Heer, Anton, British Virgin Islands (GB)
(74) Representative: Minoja, Fabrizio, Dr.

(56) References cited:
- EP-A- 0 072 868
- EP-A- 0 339 508
- EP-A- 0 481 294
- FR-A- 2 716 625
- US-A- 4 547 365

## Description

The present invention relates to mouth-soluble compositions containing N-acetylcysteine which are capable of maintaining the starting pleasant organoleptic characteristics with the passing of time.

N-Acetylcysteine is known to have a mucolytic effect promoting fluidization of the thick secretions present in the respiratory tract following both acute and chronic bronchial and pulmonary diseases.

The organoleptic characteristics of acetylcysteine restrict its oral use to pharmaceutical formulations in which the contact with mucosae occurs for a very short time, in other cases such unpleasant characteristics are masked by high amounts of flavouring and sweetening agents (for example swallowable tablets, syrups, granulates for water dissolution). Therefore, the use of mouth-soluble tablets containing N-acetylcysteine is prevented, which tablets, besides ensuring a wider, more uniform action by systemic adsorption in the gastrointestinal tract thanks to their slow dissolution rate, would also be an effective topical treatment- of the oral cavity diseases, such as stomatitis, gingivitis and the like.

EP 0 339 508 and EP 0 481 294 address the problem of providing mouth-soluble formulations containing N-acetylcysteine. According to EP 0 339 508, a sodium bicarbonate and potassium bicarbonate effervescent mixture, a carbohydrates mixture (in an about 20-50:1 ratio to N-acetylcysteine) and a flavour are used in order to mask the unpleasant taste.

EP 0 481 294 discloses solid, fast-soluble preparations in which N-acetylcysteine is mixed with cellulose and/or carboxymethylcellulose, a soluble sugar-alcohol, a sweetner and a flavouring.

FR 2716625 discloses cyclodextrins inclusion complexes of cysteamine and their use in pharmaceutical compositions for improving the stability of the active ingredient and for masking unpleasant taste and odour.

The unpleasant organoleptic characteristics of N-acetylcysteine are actually due to slight traces of a degradation product having marked sulfur taste and odour. In fact, whereas the freshly prepared product is nearly odourless and has a slightly salted, acidic taste, it becomes disgusting even after a short time.

Surprisingly, cyclodextrins have been found to be able to neutralize the unpleasant characteristics which become evident in ageing N-acetylcysteine, due to their capability of complexing a number of products, among which also N-acetylcysteine sulfur degradation products, enveloping them in the hydrophobic cavity of their molecular structure.

This fact allowed to prepare small size, high dosage mouth-soluble compositions, such as tablets and granulates, which keep unchanged their own pleasant organoleptic characteristics even in the long run.

Therefore, the present invention provides N-acetylcysteine mouth-soluble compositions which are easy to take, having the advantage of ensuring, through a slow dissolution and possibly a protracted release, uniform blood levels by systemic adsorption, thus providing a topical medicament for the oral cavity diseases which are sensitive to the treatment with N-acetylcysteine.

Moreover, compared with the compositions claimed in EP 0339 508 B1, the compositions of the invention require a smaller amount of carbohydrates-based sweetening agents necessary to mask N-acetylcysteine organoleptic characteristics: in the above cited patent, the required carbohydrates vary from 20 to 50 parts by weight compared to N-acetylcysteine; whereas, in the present invention, 2 to 15 (preferably 5 to 10) parts by weight compared to N-acetylcysteine are sufficient. This allows to formulate higher dosages of the active ingredient, which would otherwise be restricted by the amount of carbohydrates necessary, and to decrease substantially the carbohydrate dosage, particularly in diabetic diets.

Among the cyclodextrins for use in the compositions according to the invention, α-cyclodextrin, β-cyclodextrin γ-cyclodextrin and hydroxypropyl-β-cyclodextrin are preferred. Preferred carbohydrates are polyalcohols such as mannitol, sorbitol and xylitol.

The N-acetylcysteine mouth-soluble compositions of the present invention contain 50 to 1000 mg of N-acetylcysteine (preferably 100 to 600 mg), 0.2 to 4 (preferably 0.4 to 1) parts of a cyclodextrin (preferably β-cyclodextrin) and 2 to 15 (preferably 5 to 10) parts of carbohydrate-based sweetening agents to one part by weight of N-acetylcysteine, and also flavouring agents and working-up aids.

The ingredients will be combined together to prepare the pharmaceutical formulation suitable for the administration by conventional processes such as mixing, tabletting, granulation or a combination thereof.

The final compositions will be in the form of mouth-soluble tablets, mouth-soluble granulates or any other pharmaceutical formulation suitable to the administration of the active ingredient following dissolution in the oral cavity.

The following examples further illustrate the invention.

| **Example 1** | |
|---|---|
| Composition for mouth-soluble tablets | |
| N-acetylcysteine | mg 100 |
| β-cyclodextrin | mg 350 |
| Aspartame | mg 25 |
| Mint flavour | mg 50 |
| Magnesium stearate | mg 20 |
| Talc | mg 20 |
| Xylitol | mg 1435 |

For the preparation, mix together the components and press them by means of a tabletting machine.

| **Example 2** | |
|---|---|
| Composition for mouth-soluble tablets | |
| N-acetylcysteine | mg 200 |
| β-cyclodextrin | mg 100 |
| Calcium carbonate | mg 100 |
| Aspartame | mg 50 |
| Berry flavour | mg 40 |
| Magnesium stearate | mg 30 |
| Talc | mg 30 |
| Dextrose | mg 1450 |

For the preparation, mix together the components and press them by means of a tabletting machine.

### Organoleptic characteristics

Mouth-soluble tablets prepared as described above, have been stored at room temperature and at 32°C, together with similar tablets devoid of β-cyclodextrin in the formulation.

Both the tablets at room temperature and those at 32°C were checked after 12 months: cyclodextrin-containing tablets still maintained the starting pleasant organoleptic characteristics, whereas the tablets not containing cyclodextrin had, both those kept at r.t. and, to a higher degree, those at 32°C, a marked sulfur odour and an ekceedingly unpleasant taste.

| **Example 3** | |
|---|---|
| Composition for mouth-soluble tablets | |
| N-acetylcysteine | mg 200 |
| β-cyclodextrin | mg 100 |
| Calcium carbonate | mg 200 |
| Citric acid | mg 50 |
| Aspartame | mg 75 |
| Tangerine flavour | mg 60 |
| Magnesium stearate | mg 30 |
| Talc | mg 30 |
| Sorbitol | mg 1255 |

For the preparation, mix together the components and press them by means of a tabletting machine.

| **Example 4** | |
|---|---|
| Composition for mouth-soluble tablets | |
| N-acetylcysteine | mg 200 |
| β-cyclodextrin | mg 100 |
| Calcium carbonate | mg 50 |
| Aspartame | mg 50 |
| Raspberry flavour | mg 40 |
| Magnesium stearate | mg 30 |
| Talc | mg 30 |
| Sorbitol | mg 1500 |

For the preparation, mix together the components and press them by means of a tabletting machine.

| **Example 5** | |
|---|---|
| Composition for mouth-soluble granulate sachets | |
| N-acetylcysteine | mg 300 |
| Hydroxypropyl-β-cyclodextrin | mg 150 |
| Aspartame | mg 75 |
| Lemon flavour | mg 60 |
| Sorbitol | mg 4415. |

For the preparation, mix N-acetylcysteine, hydroxypropyl-β-cyclodextrin and sorbitol and granulate the mixture with depurated water in an amount of 15% on the total weight by means of fluidized bed granulator-drier.

Add aspartame and lemon flavour to the granulate, mix and distribute into sachets.

## Claims

1. Mouth-soluble compositions containing N-acetylcysteine and cyclodextrin, together with sweetening agents, flavours and working-up aids.

2. Compositions according to claim 1, wherein the N-acetylcysteine amount ranges from 50 to 1000 mg, preferably from 100 to 600 mg.

3. Compositions according to claim 1, wherein the cyclodextrin to N-acetylcysteine weight ratio is 0.2-4:1, preferably 0.4-1:1.

4. Compositions according to claim 1, wherein the cyclodextrin is α-cyclodextrin, β-cyclodextrin, γ-cyclodextrin or hydroxypropyl-β-cyclodextrin.

5. Compositions according to claim 1, wherein at least one of the sweetening agents and working-up aids is a carbohydrate.

6. Compositions according to claim 5, wherein the carbohydrate is a polyalcohol such as mannitol, sorbitol or xylitol.

7. Compositions according to claim 5, wherein the carbohydrates to N-acetylcysteine weight ratio is 2-15:1, preferably 5-10:1.

8. Compositions according to claims 1 to 7 in the form of tablets, granulates or other mouth-soluble formulations.

## Patentansprüche

1. Mundlösliche Zusammensetzungen, enthaltend N-Acetylcystein und Cyclodextrin gemeinsam mit Süßstoffen, Geschmacksstoffen, Aufarbeitungshilfsmitteln.

2. Zusammensetzungen nach Anspruch 1, wobei der N-Acetylcystein-Gehalt von 50 bis 1000 mg, bevorzugt von 100 bis 600 mg reicht.

3. Zusammensetzungen nach Anspruch 1, wobei das Gewichtsverhältnis Cyclodextrin zu N-Acetylcystein 0,2 bis 4 : 1, bevorzugt 0,4 bis 1 : 1 beträgt.

4. Zusammensetzungen nach Anspruch 1, wobei das Cyclodextrin α-Cyclodextrin, β-Cyclodextrin, γ-Cyclodextrin oder Hydroxypropyl-β-cyclodextrin ist.

5. Zusammensetzungen nach Anspruch 1, wobei mindestens eines der Süßstoffe und Aufarbeitungs-Hilfsmittel ein Kohlenhydrat ist.

6. Zusammensetzungen nach Anspruch 5, wobei das Kohlenhydrat ein Polyalkohol wie Mannitol, Sorbitol oder Xylitol ist.

7. Zusammensetzungen nach Anspruch 5, wobei das Gewichtsverhältnis Kohlenhydrate zu N-Acetylcystein 2 bis 15 : 1, bevorzugt 5 bis 10 : 1 beträgt.

8. Zusammensetzungen nach den Ansprüchen 1 bis 7 in Form von Tabletten, Granulaten oder anderen mundlöslichen Formulierungen.

## Revendications

1. Compositions solubles dans la bouche contenant de la N-acétylcystéine et de la cyclodextrine, associées à des agents édulcorants, des arômes et des aides de fabrication.

2. Compositions selon la revendication 1 dans lesquelles la quantité de N-acétylcystéine se situe dans l'intervalle de 50 à 1000 mg, de manière préférable de 100 à 600 mg.

3. Compositions selon la revendication 1 dans lesquelles le rapport en poids de la cyclodextrine à la N-acétylcystéine est de 0,2 à 4:1, de manière préférable, de 0,4 à 1:1.

4. Compositions selon la revendication 1 dans lesquelles la cyclodextrine est l'α-cyclodextrine, la β-cyclodextrine, la γ-cyclodextrine ou l'hydroxypropyl-β-cyclodextrine.

5. Compositions selon la revendication 1 dans lesquelles au moins un des agents édulcorants et des aides de fabrication est un hydrate de carbone.

6. Compositions selon la revendication 5 dans lesquelles l'hydrate de carbone est un polyalcool tel que le mannitol, le sorbitol ou le xylitol.

7. Compositions selon la revendication 5 dans lesquelles le rapport en poids des hydrates de carbone à la N-acétylcystéine est de 2 à 15:1, de manière préférable, de 5 à 10:1.

8. Compositions selon les revendications 1 à 7 sous forme de comprimés, granulés ou d'autres formulations solubles dans la bouche.
